(19) Europäisches Patentamt
European Patent Office
Office européen des brevets

(11) Veröffentlichungsnummer : **0 196 272 B1**

# (12) EUROPÄISCHE PATENTSCHRIFT

(45) Veröffentlichungstag der Patentschrift : **07.06.89**

(51) Int. Cl.4 : **C 07 C147/06**, C 07 C121/70

(21) Anmeldenummer : **86810074.4**

(22) Anmeldetag : **10.02.86**

## (54) Neue Sulfonverbindungen.

(30) Priorität : **15.02.85 CH 730/85**

(43) Veröffentlichungstag der Anmeldung : **01.10.86 Patentblatt 86/40**

(45) Bekanntmachung des Hinweises auf die Patenterteilung : **07.06.89 Patentblatt 89/23**

(84) Benannte Vertragsstaaten : **CH DE FR GB LI**

(56) Entgegenhaltungen : **EP-A- 0 041 043**

(73) Patentinhaber : **CIBA-GEIGY AG**
**Klybeckstrasse 141**
**CH-4002 Basel (CH)**

(72) Erfinder : **Burdeska, Kurt, Dr.**
**Laufenburgerstrasse 30**
**CH-4058 Basel (CH)**
Erfinder : **Weber, Kurt, Dr.**
**Rennweg 98**
**CH-4052 Basel (CH)**
Erfinder : **Reinehr, Dieter, Dr.**
**Wolfsheule 10**
**D-7842 Kandern (DE)**

**EP 0 196 272 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Sulfonverbindungen, ein Verfahren zu deren Herstellung, deren Verwendung als Zwischenprodukte für optische Aufheller und neue asymmetrisch substituierte Stilbenverbindungen.

Die neuen Sulfonverbindungen entsprechen der Formel I

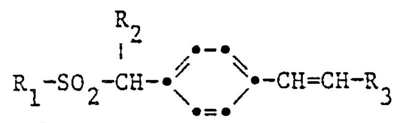

(I)

worin bedeutet :

$R_1$ einen gegebenenfalls substituierten Phenyl- oder Naphthylrest,

$R_2$ Wasserstoff oder den Rest der Formel

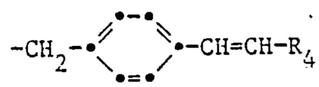

$R_3$ und $R_4$ —CN oder —$COOR_5$ mit $R_5$ $C_1$-$C_4$-Alkyl, wobei für den Fall, dass $R_2$ den Rest der Formel

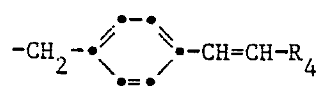

bedeutet $R_3$ und $R_4$ in Formel I gleich oder voneinander verschieden sein können.

Bedeutet $R_1$ einen gegebenenfalls substituierten Phenyl- oder Naphthylrest, so kommen als Substituenten beispielsweise in Frage : Halogen, wie Fluor, Chlor oder Brom ; $C_1$-$C_4$-Alkyl (verzweigt und unverzeigt) wie Methyl, Aethyl, n- und iso-Propyl, sowie $NO_2$ und Phenyl.

In den bevorzugten Verbindungen der Formel I bedeutet $R_1$ einen gegebenenfalls substituierten Phenylrest, vorallem einen durch $C_1$-$C_4$-Alkyl insbesondere Methyl, substituierten Phenylrest.

$R_2$ stellt in den bevorzugten Verbindungen den Rest der Formel

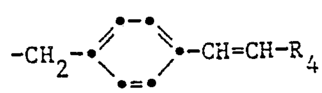

dar.

$R_4$ in der Bedeutung einer $COOR_5$-Gruppe stellt vorallem folgende Gruppen dar : —$COOCH_3$, —$COOC_2H_5$, —$COOC_3H_7$(n) und —$COOC_4H_9$(n). In bevorzugten Verbindungen bedeutet $R_4$CN.

Für den Fall, dass $R_2$ die Gruppierung

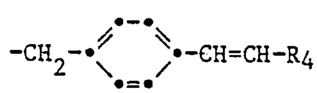

darstellt so können die beiden Substituenten $R_3$ und $R_4$ gemäss der Formel I im Rahmen der Definition gleich (z. B. zweimal CN) oder voneinander verschieden sein. (z. B. $R_3$ bedeutet CN und $R_4$ stellt die $COOR_5$ Gruppe dar.)

Die neuen Sulfonverbindungen der Formel I worin $R_2$ die Gruppe der Formel

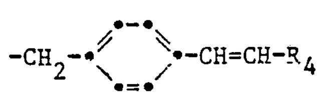

darstellt sind vorallem wertvolle Zwischenprodukte für die Gewinnung von optischen Aufhellern der Stilbenreihe. Diejenigen Verbindungen der Formel I worin $R_2$ Wasserstoff bedeutet dienen vorallem zur Herstellung derjenigen Verbindungen der Formel I worin $R_2$ die Gruppe der Formel

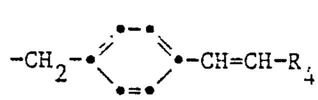

bedeutet.

Die Sulfonverbindungen der Formel I werden z. B. hergestellt, indem man ein Zimtsäurederivat der Formel II

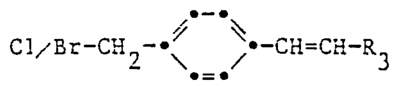 (II)

worin $R_3$ die angegebene Bedeutung hat mit dem Natriumsalz der Benzol- oder Naphthalinsulfinsäure der Formel $R_1$—$SO_2NA$ (III) in organischem, vorallem alkalischem Medium zu einer Verbindung der Formel Ia

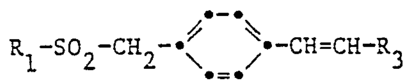 (Ia)

umsetzt, und diese gegebenenfalls mit einem weiteren Mol eines gleichen oder verschiedenen Zimtsäurederivates der Formel IV

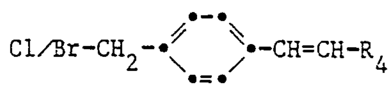 (IV)

reagieren lässt.

Die Umsetzung des Zimtsäurederivates II mit der Verbindung III erfolgt im organischem Medium, z. B. in Dimethylformamid, Dimethylsulfoxyd oder Tetrahydrofuran, vorallem jedoch im alkoholischen Medium in der Hitze. Geeignete Alkohole sind beispielsweise: Methanol, Aethanol, Propanol, Isopropanol, Butanole, Glykole und Glykoläther.

Die Verbindungen II, III und IV sind bekannt und können nach bekannter Art und Weise erhalten werden. Als Zimtsäurederivate II bzw. IV kommen beispielsweise in Frage:

4-Brommethylzimtsäurenitril,
4-Chlormethylzimtsäurenitril,
4-Brommethylzimtsäuremethylester, und
4-Brommethylzimtsäureäthylester.

Beispiele für die Ausgangsverbindungen III sind beispielsweise:

Benzolsulfinsäure (Na-Salz),
4-Methylbenzolsulfinsäure (Na-Salz),
4-Chlorbenzolsulfinsäure (Na-Salz), und
β-Naphthylsulfinsäure (Na-Salz).

Man erhält so die Verbindungen der Formel I worin $R_2$ Wasserstoff bedeutet (Formel Ia). Diese Verbindungen dienen zur Herstellung der Verbindungen der Formel I worin $R_2$ die Gruppe der Formel

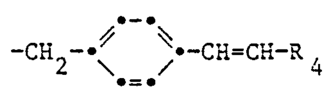

darstellt, indem man die Verbindungen der Formel Ia mit einem weiteren Mol eines Zimtsäurederivates der Formel IV umsetzt. Diese Umsetzung erfolgt unter Zuhilfenahme einer Base (z. B. NaOH) in einem organischen Lösungsmittel. Vor allem verwendet man solche Lösungsmittel, die sich für eine Phasentransferreaktion eignen, wie Methylenchlorid oder Chlorbenzol; es kommen aber auch Mischungen derartiger Lösungsmittel, z. B. mit Dimethylformamid oder Dimethylsulfoxid in Frage. Die Reaktion wird gegebenenfalls unter Phasentransferbedingungen (Zuhilfenahme eines Phasentransferkatalysators wie Tetrabutylammoniumbromid, Butyltriäthylammoniumbromid oder -chlorid, Benzyltributylammoniumbromid oder -chlorid) durchgeführt.

Verwendung finden die neuen Sulfonverbindungen der Formel I, worin $R_2$ die Gruppe der Formel

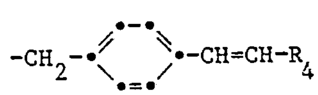

bedeutet, zur Herstellung von Divinylstilbenverbindungen, darunter als neue Verbindungen die unsymmetrisch substituierten 4,4'-Divinylstilbenverbindungen der Formel

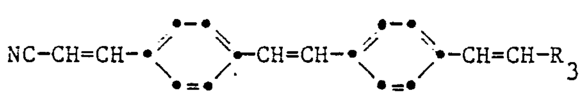 (V)

worin $R_3$—$COOR_5$ und $R_5$ darin $C_{1-4}$-Alkyl bedeuten.

Diese Stilbenverbindungen werden zweckmässigerweise derart erhalten, dass man die genannten Verbindungen der Formel I worin $R_2$ die Gruppe der Formel

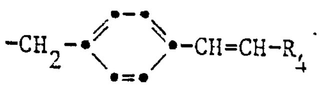

bedeutet, in Wasser oder in einem organischen Lösungsmittel (beispielsweise niedere Alkohole wie Methanol, Aethanol, Propanol, Isopropanol oder Butanol, Glykole, Glykolmonoalkyläther, Polyglykolalkyläther, Pyridin, N-Methylpyrridon, aromatische Kohlenwasserstoffe und insbesondere Dimethylformamid oder Dimethylsulfoxid) oder Gemischen davon löst oder suspendiert, mit einer Base versetzt und auf ca. 40-150 °C erhitzt.

Als Basen kommen anorganische und organische Verbindungen, wie Alkalien, Alkoholate oder auch Amine in Frage. Beispielsweise sind genannt: NaOH, $Na_2CO_3$, KOH, $K_2CO_3$, LiOH, $CaCO_3$, Triäthylamin und Pyridin.

Die aus den neuen Sulfonverbindungen hergestellten Stilbenverbindungen eignen sich als optische Aufheller für Polyesterfasern.

Die folgenden Beispiele veranschaulichen die Erfindung ohne sie darauf zu limitieren. Die Temperaturen sind in Grad Celsius angegeben.

Beispiel 1

44,4 g 4-Brommethylzimtsäurenitril werden mit 36,11 g des Natriumsalzes der Benzolsulfinsäure und 350 ml Aethanol für 3 1/2 Stunden zum Sieden erhitzt. Das nach dem Abkühlen auf Raumtemperatur ausgefallene Produkt wird abfiltriert, mit Aethanol und Wasser gewaschen und im Vakuum bei 80° getrocknet. Man erhält 52 g des Produktes der Formel

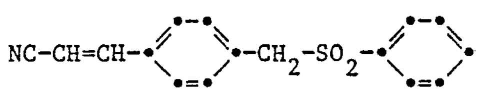

vom Schmelzpunkt 181-182°.

Verwendet man anstelle der 44,4 g 4-Brommethylzimtsäurenitril 35,53 g 4-Chlormethylzimtsäurenitril (Schmelzpunkt 85-86°) so erhält man, bei sonst gleicher Arbeitsweise, das 4-Cyanvinylbenzylphenylsulfon in einer Ausbeute von 87 % der Theorie.

Analog zum Beispiel 1 können die in Tabelle 1 aufgeführten Verbindungen hergestellt werden:

Tabelle 1

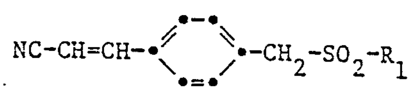

| Beispiel | $R_1$ | Schmelzpunkt °C |
|---|---|---|
| 2 | 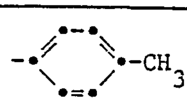 | 179 - 181° |
| 3 | 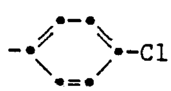 | 218 - 219° |
| 4 | 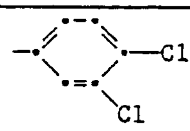 | 219 - 220° |
| 5 | 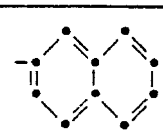 | 193 - 194° |
| 6 | 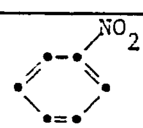 | 187-188° |

Beispiel 7

38,26 g 4-Brommethylzimtsäuremethylester (Schmelzpunkt 59-60°) werden mit 27,9 g des Natriumsalzes der Benzolsulfinsäure und 200 ml Aethanol für drei Stunden am Rückfluss zum Sieden erhitzt. Nach dem Abkühlen auf Raumtemperatur wird das ausgefallene Produkt abfiltriert, mit Aethanol und Wasser gewaschen und getrocknet. Man erhält 43,4 g (91,6 %) der Verbindung der Formel

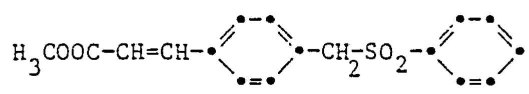

vom Schmelzpunkt 162-163°.

Verwendet man anstelle des 4-Brommethylzimtsäuremethylesters äquivalente Mengen des 4-Brommethylzimtsäureäthylester (Schmelzpunkt 45-47°) so erhält man, bei sonst gleicher Arbeitsweise die Verbindung der Formel

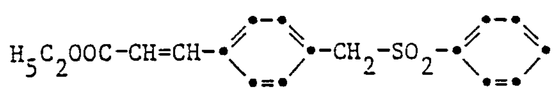

vom Schmelzpunkt 137-138°.

Durch Umsatz des 4-Brommäthylzimtsäuremethylesters mit dem Natriumsalz der 4-Chlorbenzolsulfinsäure wird die Verbindung

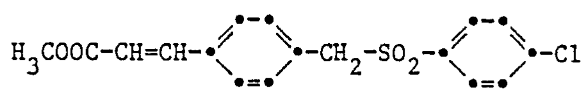

mit einem Schmelzpunkt von 227-228 °C erhalten.

Beispiel 8

28,33 g 4-Cyanvinylbenzyl-phenylsulfon, hergestellt nach Beispiel 1, und 22,2 g Brommethylzimtsäurenitril werden in einem Gemisch von 200 ml Methylenchlorid und 40 ml Dimethylsulfoxyd gelöst. Nach Zugabe von 2 g Tetrabutylammoniumbromid und 14 g einer 30 %igen wässrigen Natriumhydroxydlösung wird unter schnellem Rühren auf Rückfluss erhitzt und 12 Stunden am Sieden gehalten. Nach 5 Stunden werden dem Reaktionsgemisch nochmals 0,5 g Tetrabutylammoniumbromid zugegeben.

Nach Beendigung der Reaktion wird das Methylenchlorid im Vakuum abdestilliert und der Rückstand mit 200 ml Wasser versetzt. Das angefallene Produkt wird abfiltriert, mit Wasser fein gemahlen, nochmals filtriert, mit Wasser und dann mit Aethanol gewaschen und im Vakuum bei 80° getrocknet. Man erhält 42,1 g (97,1 % der Theorie) des Produktes der Formel

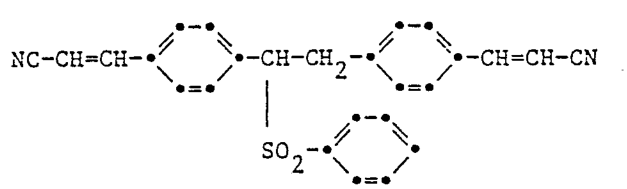

mit einem Schmelzpunkt von 200-202°.

Analog zum Beispiel 8 können die in Tabelle 2 aufgeführten Verbindungen hergestellt werden :

(Siehe Tabelle 2 Seite 6 f.)

Tabelle 2

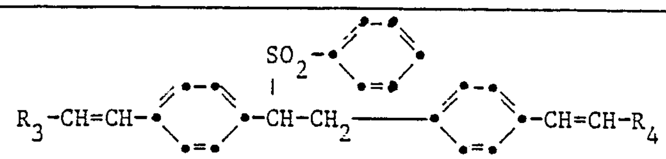

| Beispiel | $R_3$ | $R_4$ | Schmelzpunkt °C |
|---|---|---|---|
| 9 | $-CN$ | $-COOCH_3$ | 156-157° |
| 10 | $-CN$ | $-COOC_2H_5$ | 166-168° |
| 11 | $H_3COOC$ | $-COOCH_3$ | 132-133° |
| 12 | $H_5C_2OOC$ | $-COOC_2H_5$ | 140-141° |
| 13 | $H_3COOC$ | $-COOC_2H_5$ | 135-136° |

Beispiel 14

21,22 g der nach Beispiel 8 hergestellten Verbindung werden in 140 ml Aethylenglykol suspendiert und nach Zugabe von 5,18 g wasserfreiem Kaliumkarbonat unter gutem Rühren für 4 Stunden auf 100-105° erhitzt. Man lässt danach auf Raumtemperatur abkühlen und versetzt das Reaktionsgemisch vorsichtig mit 6 ml Essigsäure. Das angefallene Gemisch wird noch 20 Minuten bei Raumtemperatur gerührt, danach abfiltriert und nacheinander mit Methanol, Wasser und Methanol gewaschen und bei 80° im Vakuum getrocknet. Man erhält 14 g der Stilbenverbindung der Formel

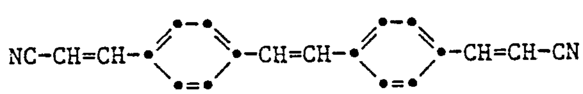

in Form von hellgrünen Kristallen vom Schmelzpunkt 222-225°.

Beispiel 15

27,45 g der nach Beispiel 9 hergestellten Verbindung werden in 90 ml Dimethylformamid gelöst und bei Raumtemperatur innerhalb von 30 Minuten mit 11 g einer 30 %igen Natriummethylatlösung versetzt. Anschliessend wird für weitere 30 Minuten bei Raumtemperatur und zur Beendigung der Reaktion noch 1 Stunde bei 40-45 °C gerührt. Nach Zusatz von 10 ml Methanol wird auf 5 °C abgekühlt, das ausgefallene Produkt der Formel

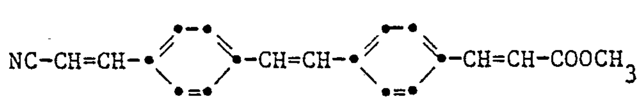

abfiltriert, mit Methanol und Wasser gewaschen und bei 80 °C im Vacuum getrocknet. Die Ausbeute beträgt 13,7 g grünlich-gelber Kristalle vom Schmelzpunkt 183-186 °C.

Beispiel 16

Bei Verwendung der Verbindung von Beispiel 10 wird die folgende Verbindung erhalten :

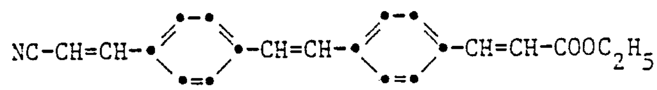

Schmelzpunkt 151-153 °C.

## Patentansprüche

1. Sulfonverbindungen der Formel I

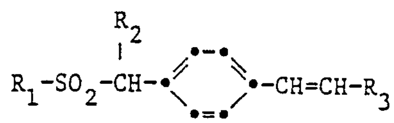

(I)

worin bedeutet :

$R_1$ einen gegebenenfalls substituierten Phenyl- oder Naphthylrest,

$R_2$ Wasserstoff oder den Rest der Formel

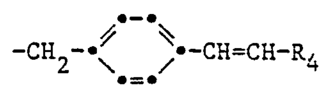

$R_3$ und $R_4$ —CN oder —$COOR_5$ mit $R_5$ $C_1$-$C_4$-Alkyl, wobei für den Fall, dass $R_2$ den Rest der Formel

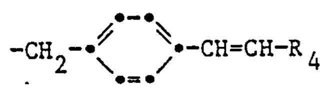

bedeutet, $R_3$ und $R_4$ in Formel I gleich oder voneinander verschieden sein können.

2. Sulfonverbindungen gemäss Anspruch 1 worin $R_1$ einen gegebenenfalls substituierten Phenylrest bedeutet.

3. Sulfonverbindungen gemäss Anspruch 2 worin $R_1$ einen gegebenenfalls durch $C_1$-$C_4$-Alkyl substituierten Phenylrest bedeutet.

4. Sulfonverbindungen gemäss Anspruch 1 worin $R_2$ den Rest der Formel

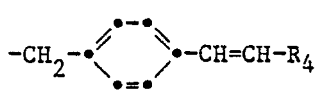

bedeutet.

5. Sulfonverbindungen gemäss Anspruch 1 worin $R_3$ und $R_4$ CN bedeuten.

6. Verfahren zur Herstellung von Sulfonverbindungen der Formel I gemäss Anspruch 1, dadurch gekennzeichnet, dass man ein Zimtsäurederivat der Formel II

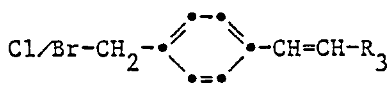

(II)

worin $R_3$ die im Anspruch 1 angegebene Bedeutung hat mit dem Natriumsalz der Benzol- oder Naphthalinsulfinsäure der Formel $R_1$—$SO_2Na$ (III) zu einer Verbindung der Formel Ia

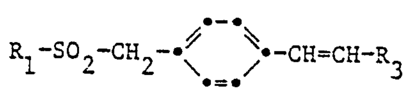

(Ia)

umsetzt, und diese gegebenenfalls mit einem weiteren Mol eines gleichen oder verschiedenen Zimtsäurederivates der Formel IV

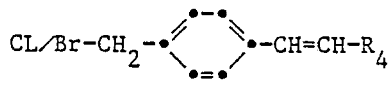

(IV)

reagieren lässt.

7. Verwendung der Sulfonverbindungen gemäss Anspruch 1 zur Herstellung von optischen Aufhellern der Stilbenreihe, dadurch gekennzeichnet, dass man Verbindungen der Formel I, worin $R_2$ die Gruppe der Formel

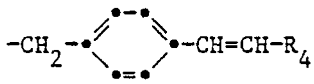

bedeutet, in einem wässrigen oder organischen Lösungs- oder Suspensionsmittel mit einer organischen oder anorganischen Base versetzt und durch Erhitzen auf eine Temperatur zwischen 40 und 150 °C die aromatische Sulfongruppe abspaltet.

## Claims

1. A sulfone compound of the formula I

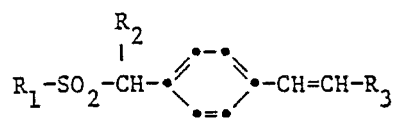

(I)

wherein

$R_1$ is an unsubstituted or substituted phenyl or naphthyl radical,

$R_2$ is hydrogen or the radical of the formula

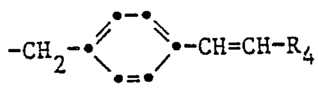

$R_3$ and $R_4$ are —CN or —$COOR_5$, in which $R_5$ is $C_1$-$C_4$-alkyl, where if $R_2$ is the radical of the formula

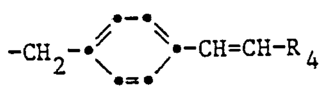

then $R_3$ and $R_4$ in formula I may be identical or different from one another.

2. A sulfone compound according to claim 1, wherein $R_1$ is an unsubstituted or substituted phenyl radical.

3. A sulfone compound according to claim 2, wherein $R_1$ is a phenyl radical which is unsubstituted or substituted by $C_1$-$C_4$-alkyl.

4. A sulfone compound according to claim 1, wherein $R_2$ is the radical of the formula

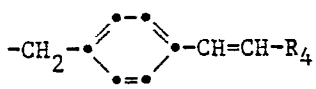

5. A sulfone compound according to claim 1, wherein $R_3$ and $R_4$ are —CN.

6. A process for the preparation of a sulfone compound of the formula I according to claim 1, which process comprises reacting a cinnamic acid derivative of the formula II

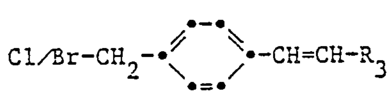

(II)

wherein $R_3$ is as defined in claim 1, with the sodium salt of the benzenesulfinic or naphthalenesulfinic acid of the formula $R_1$—$SO_2Na$ (III) to give a compound of the formula Ia

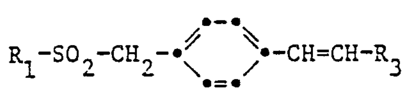

(Ia)

and, if desired, allowing this compound to react with one further mole of an identical or different cinnamic acid derivative of the formula IV

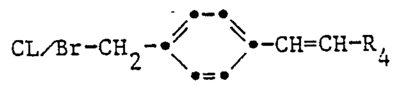

(IV)

7. Use of a sulfone compound according to claim 1 for the preparation of fluorescent whitening agents of the stilbene series, which comprises adding an organic or inorganic base to a compound of the formula I, wherein $R_2$ is the group of the formula

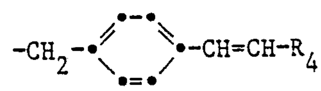

in an aqueous or organic solvent or suspending agent, and removing the aromatic sulfone group by heating to a temperature between 40 and 150 °C.

**Revendications**

1. Composés sulfones de formule I

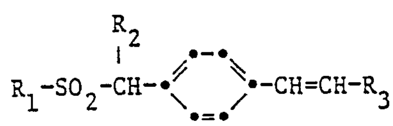

(I)

où

$R_1$ représente un reste phényle ou naphtyle éventuellement substitué.
$R_2$ représente un hydrogène ou le reste de formule

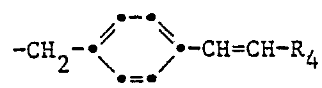

$R_3$ et $R_4$ représentent —CN ou —$COOR_5$ avec $R_5$ représentant un groupe alkyle en $C_1$-$C_4$, $R_3$ et $R_4$ pouvant être identiques ou différents dans la formule I lorsque $R_2$ représente le reste de formule

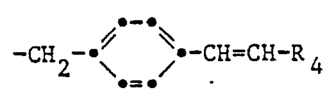

2. Composés sulfones selon la revendication 1, où $R_1$ représente un reste phényle éventuellement substitué.

3. Composés sulfones selon la revendication 2, où $R_1$ représente un reste phényle éventuellement substitué par un groupe alkyle en $C_1$-$C_4$.

4. Composés sulfones selon la revendication 1, où $R_2$ représente le reste de formule

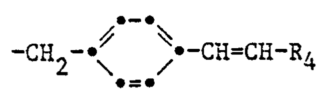

5. Composés sulfones selon la revendication 1, où $R_3$ et $R_4$ représentent CN.

6. Procédé de fabrication des composés sulfones de formule I selon la revendication 1, caractérisé en ce que l'on fait réagir un dérivé de l'acide cinnamique de formule II

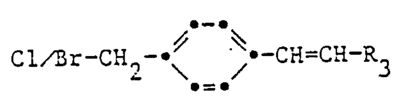

(II)

où $R_3$ a la même signification que dans la revendication 1, avec le sel de sodium de l'acide benzène- ou naphtalène-sulfinique de formule $R_1$—$SO_2Na$ (III) pour obtenir un composé de formule Ia

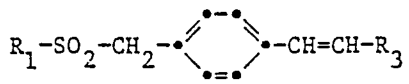 (Ia)

et que l'on fait réagir celui-ci éventuellement avec une autre mole d'un dérivé identique ou différent de l'acide cinnamique, de formule IV

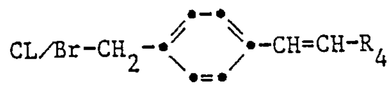 (IV)

7. Utilisation des composés sulfones selon la revendication 1 pour la fabrication d'azurants optiques de la série des stilbènes, caractérisée en ce qu'aux composés de formule I, où $R_2$ représente le groupe de formule

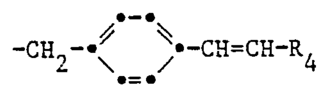

on ajoute, dans un solvant ou un milieu de suspension aqueux ou organique, une base organique ou minérale et que, par chauffage à une température comprise entre 40 et 150 °C, on détache le groupe sulfone aromatique.